(19) **Europäisches Patentamt / European Patent Office / Office européen des brevets**

(11) **EP 2 814 848 B1**

(12) **EUROPEAN PATENT SPECIFICATION**

(45) Date of publication and mention
of the grant of the patent:
**06.09.2017 Bulletin 2017/36**

(51) Int Cl.:
***C08B 37/00*** *(2006.01)*

(21) Application number: **13704450.9**

(22) Date of filing: **15.02.2013**

(86) International application number:
**PCT/EP2013/053087**

(87) International publication number:
**WO 2013/121001 (22.08.2013 Gazette 2013/34)**

(54) **PARTIALLY DEPOLYMERIZED GLYCOSAMINOGLYCAN SILVER AND GOLD SALTS**

TEILWEISE DEPOLYMERISIERTE SILBER- UND GOLDSALZE VON GLYCOSAMINOGLYCANEN

SELS DE L'ARGENT ET DE L'OR DES GLYCOSAMINOGLYCANES PARTIELLEMENT DÉPOLYMÉRISÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2012 ES 201230247**

(43) Date of publication of application:
**24.12.2014 Bulletin 2014/52**

(73) Proprietor: **Kimflexor, S.L.**
**08303 Mataró (ES)**

(72) Inventors:
• **FLORES I SALGADO, Francesc**
**E-08303 Mataró (ES)**

• **BENÍTEZ JIMÉNEZ, Antonio, Francisco**
**E-08397 Pineda De Mar (ES)**
• **COSTA I RIEROLA, Margarita**
**E-08303 Mataró (ES)**
• **FLORES I COSTA, Roger**
**E-08303 Mataró (ES)**
• **FLORES I COSTA, Laia**
**E-08303 Mataró (ES)**

(74) Representative: **ZBM Patents - Zea, Barlocci & Markvardsen**
**Plaza Catalunya, 1**
**08002 Barcelona (ES)**

(56) References cited:
**CN-A- 1 687 141      US-A1- 2010 317 617**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to the field of pharmacy and cosmetics; in particular, it relates to partially depolymerized glycosaminoglycan metal salts. It also relates to a process for the preparation of said salts, to oral and topical pharmaceutical and cosmetic compositions containing them, and to their pharmaceutical and cosmetic uses.

BACKGROUND ART

It is known the use of noble metals as antimicrobial agents, in particular the use of simple silver salts, mainly in the prophylaxis and the topical treatment of superficial infections, especially pathogens. However, the application of the silver salts has been limited due to their inherent structural instability and/or photosensitivity, leading to storage difficulties and/or to a darkening of color, which is a considerable aesthetic drawback and limits their cosmetic and medical application.

Silver glycosaminoglycan derivatives, among other metals are also known. For example, document US 2010317617 describes a composite material comprising silver nanoparticles conjugated with a glycosaminoglycan or glucose, where the silver nanoparticles are embedded within the surrounding glycosaminoglycan matrix. Said nanoparticles can be used as antimicrobial agents by topical application on wounds or burns, and also for coating plastic, catheters or surgical instruments.

Document WO 8705517 describes heavy metal salts (including silver, gold, cerium and tungsten) of hyaluronic acid for their use in the inhibition of microbial growth.

Finally, it is also known from document CN 1687141 that silver chondroitin sulfate can be used for the treatment of burns. The silver content in the salt described in this document is 19-33%, the sodium content is lower than 0.5% and the nitrate content lower than 100 ppm. Further, document EP 1878754 describes the use of the heparin silver salt and its use for the treatment of burns. The silver content in the salt described in this document is 25-45%, the sodium content is lower than 2.5% and the nitrate content lower than 0.1%.

However, the glycosaminoglycan silver and gold derivatives described in the previous documents do not have suitable light stability. Therefore, there is a need to find products derived from glycosaminoglycans which are stable and have high antimicrobial activity.

SUMMARY OF THE INVENTION

The inventors have found that the partially depolymerized glycosaminoglycan (GAG) metal salts, obtainable by the process of the invention which comprises the steps of chemical depolymerization, bleaching and ion exchange, show good antimicrobial activity and are stable when exposed to light, unlike other similar high molecular weight non-depolymerized salts.

This improvement in the stability of the salts of the invention is demonstrated in the examples, where the light stability of partially depolymerized silver chondroitin sulfate, prepared by the process of the present invention, is compared to non-depolymerized silver chondroitin sulfate, such as for example the one described in document CN 1687141. The results show that partially depolymerized silver chondroitin sulfate is stable under exposure to ambient light unlike the non-depolymerized product.

Moreover, the topical application of the salts of the present invention does not have the adverse effects of other known silver salts such as for example silver nitrate, and, additionally is also useful for the treatment of a previously existing burn, as demonstrated in the examples. Thus, while the use of silver nitrate produces irritation, itching and burning when it is applied on the skin, the topical application of the partially depolymerized glycosaminoglycan salts of the present invention is useful to heal a burn caused by silver nitrate.

In addition, the partially depolymerized glycosaminoglycan salts of the present invention also have the advantage that they possess lower viscosity, in particular, with respect to the non-depolymerized products, and as a consequence, better absorption. Said better absorption could lead to greater efficacy of the product, such that the dose of product needed could be reduced.

The partially depolymerized glycosaminoglycan salts of the present invention can be defined by their process of preparation. Both the salts and the process for their preparation are considered part of the invention and are explained in detail below.

Therefore, one aspect of the present invention relates to a process for the preparation of a partially depolymerized glycosaminoglycan metal salt, wherein the metal is gold or silver, which comprises the following steps:

a) depolymerization of a high molecular weight glycosaminoglycan metal salt; wherein the metal is different from gold or silver, with a depolymerizing agent selected from the group consisting of potassium permanganate, sodium hydroxide, and hydrogen peroxide;

a') depolymerization of the product obtained in step a) with a depolymerizing agent selected from the group consisting of potassium permanganate, sodium hydroxide, and hydrogen peroxide;

a") depolymerization of the product obtained in step a') with a depolymerizing agent selected from the group consisting of potassium permanganate, sodium hydroxide, and hydrogen peroxide;

wherein the depolymerizing agent of step a) is potassium permanganate, the depolymerizing agent of step a') is sodium hydroxide, and the depolymerizing agent of step a") is hydrogen peroxide;b) bleaching of the product obtained in the previous step with hydrogen peroxide; and

c) metal ion exchange of the product obtained in the previous step for gold or silver by using a source of gold or silver.

The starting high molecular weight glycosaminoglycan metal salt may be commercially available or may be obtained by processes of extraction and purification of organic tissues, for example from cartilaginous tissues, where the highest concentration of GAGs can be found. These can be bovine, porcine trachea, chicken, duck, cow or pork nose, cow scapula, birds legs, shark fins-head-spine-tail, pig or cow intestinal mucosa (in the case of heparin), chicken crest and pork skin. Other possible sources of GAGs are eyes, veins, umbilical cords, etc. Said processes of extraction and purification of the high molecular weight glycosaminoglycan metal salts are well known to the person skilled in the art.

The metal of the high molecular weight glycosaminoglycan salt is different from gold and silver, and may be sodium, potassium, calcium, zinc, magnesium, iron, among others, preferably sodium. The glycosaminoglycan may be chondroitin sulfate, hyaluronic acid, dermatan sulfate, keratan sulfate, heparin or heparan sulfate, preferably, chondroitin sulfate or hyaluronic acid. In a preferred embodiment, the high molecular weight glycosaminoglycan metal salt is selected from the group consisting of sodium chondroitin sulfate and sodium hyaluronate.

The expression "high molecular weight glycosaminoglycan" refers to the purified glycosaminoglycan that has not undergone any depolymerization process. For the purposes of the present invention, the expressions "high molecular weight glycosaminoglycan" and "non-depolymerized glycosaminoglycan" are used interchangeably.

The molecular weight of the high molecular weight glycosaminoglycan metal salt will depend on the type of glycosaminoglycan and the type of metal used. For example, when the starting glycosaminoglycan is chondroitin sulfate, the molecular weight of the non-depolymerized salt is comprised from 10.000-50.000 Daltons; when the starting glycosaminoglycan is hyaluronic acid, the molecular weight of the non-depolymerized salt is comprised from 50.000-2.500.000 Daltons; when the starting glycosaminoglycan is dermatan sulfate, the molecular weight of the non-depolymerized salt is comprised from 20.000-30.000 Daltons; when the starting glycosaminoglycan is keratan sulfate, the molecular weight of the non-depolymerized salt is comprised from 5.000-10.000 Daltons; and when the starting glycosaminoglycan is heparin or heparan, the molecular weight of the non-depolymerized salt is comprised from 3.000-30.000 Daltons.

In a preferred embodiment, the high molecular weight glycosaminoglycan metal salt is a sodium chondroitin sulfate salt having a molecular weight comprised from 10.000-50.000 Daltons or a sodium hyaluronate having a molecular weight comprised from 50.000-2.500.000 Daltons.

The depolymerization process of the invention comprises three depolymerization steps, wherein the depolymerizing agents are selected from potassium permanganate, sodium hydroxide and hydrogen peroxide; one bleaching step and one metal ion-exchange step. These steps can be carried out consecutively, or, after each of them, a purification step can be optionally carried out, which can include the isolation of the product.

An advantage of the depolymerization process which comprises the three steps mentioned above is that it gives rise to products that maintain the properties of the non-depolymerized products without being degraded, i.e. the depolymerized products maintain their properties such as for example, moisturizing and anti-inflammatory properties, wound healing, anticoagulant, symptomatic treatment of osteoarthritis/osteoporosis, etc.

Although the invention is especially related to a chemical depolymerization which comprises the three steps mentioned above, the depolymerization could also be carried out through other oxidative processes or with enzymes specific for each glycosaminoglycan, such as chondroitinases, hyaluronidases, heparinases, etc.

The depolymerization step a) with potassium permanganate comprises reacting a high molecular weight glycosaminoglycan salt, wherein the metal is different from gold or silver, in a suitable solvent, preferably water.

In a preferred embodiment, the amount of potassium permanganate is comprised from 5-30% by weight of the amount by weight of starting non-depolymerized glycosaminoglycan salt, more preferably between 10-25%, and more preferably 20%. In another preferred embodiment, this reaction is carried out at a pH comprised from 7-10, more preferably from 8-9, and more preferably 8.5. In another preferred embodiment, the reaction is carried out by heating, preferably at a temperature comprised from 60-95 °C, more preferably from 70-95 °C, and more preferably at 80 °C. In another preferred embodiment, the reaction is carried out during a suitable period of time, preferably comprised from 0.5-2 h, more preferably for 1 h.

Optionally, the solution obtained after the depolymerization step with potassium permanganate can be filtered to remove solid impurities.

The depolymerization step a') with sodium hydroxide comprises reacting the product obtained in step a) in a suitable solvent, preferably water.

In a particular embodiment, the sodium hydroxide used is aqueous, preferably at 30% (weight/volume). In another preferred embodiment, the amount of sodium hydroxide is the amount necessary to adjust the pH at a value comprised from 8-11, more preferably from 9-11.

In a preferred embodiment, the depolymerization step a') with sodium hydroxide is carried out in the presence of an inorganic salt, such as sodium or potassium chloride. In a more preferred embodiment, the inorganic salt is sodium chloride. In a more preferred embodiment, the amount of sodium chloride is comprised from 10-20% by weight of the amount by weight of starting non-depolymerized glycosaminoglycan salt, more preferably from 13-17%, more preferably 15%. In another preferred embodiment, the reaction is carried out by heating, preferably at a temperature comprised from 35-55 °C, more preferably from 40-50 °C, more preferably 45 °C. In another preferred embodiment, the reaction is carried out for a period of time comprised from 6-15 h, more preferably from 10-12 h.

The depolymerization step a") with hydrogen peroxide comprises reacting the product obtained in step a') in a suitable solvent, preferably water.

When the product of step a) is not isolated and the product is in solution, step a') may be carried out in the same solvent of step a), adjusting the pH, if necessary. When the product of step a') is not isolated and the product is in solution, step a") may be carried out in the same solvent of step a'), adjusting the pH, if necessary.

In a preferred embodiment, the amount of hydrogen peroxide in the depolymerization step is comprised from 5-25% by weight of the amount by weight of starting non-depolymerized glycosaminoglycan salt, more preferably from 10-20 %, and more preferably 15%. In another preferred embodiment, this reaction is carried out at a pH comprised from 9-11, more preferably from 10-11. In another preferred embodiment, the reaction is carried out at a suitable temperature, preferably from 20-35 °C, more preferably from 25-30 °C. In a particular embodiment, the reaction is carried out for a period of time comprised from 0.5-2 h, more preferably 1-2 h.

The depolymerized product of step a), a') or a") can be optionally purified before the bleaching step. Said purification may comprise the filtration of the solution obtained in the previous step to remove solid impurities. Additionally or alternatively, the purification may include the precipitation of the product using organic solvents and isolation of the same. In a preferred embodiment, the precipitation is carried out at a pH comprised from 5.0-7.5, more preferably from 5.5-6.0 The adjustment of pH may be carried out by the addition of an acid as e.g. acetic acid, hydrochloric acid or sulphuric acid. The organic solvent can be, among others, methanol, ethanol, acetone or mixtures thereof. The isolation of the partially depolymerized glycosaminoglycan gold or silver salt, may be carried out e.g. by filtration, centrifugation or decantation. In a particular embodiment, the organic solvent used for the precipitation of the salt is ethanol or methanol.

The bleaching step is carried out by reacting the product obtained in the previous step with hydrogen peroxide in a suitable solvent, preferably water. When the product of the previous step is not isolated and the product is in solution, step b) may be carried out in the same solvent of the previous step, adjusting the pH, if necessary.

In a preferred embodiment, the amount of hydrogen peroxide in the bleaching step is comprised from 45-60% by weight of the amount by weight of starting non-depolymerized glycosaminoglycan salt, more preferably from 50-60%, and more preferably 50%. In another preferred embodiment, this reaction is carried out at a pH comprised from 5.0-7.5, more preferably from 5.5-6.0. In a particular embodiment, the reaction is carried out at a suitable temperature, preferably from 20-45 °C, more preferably from 30-45 °C. In another particular embodiment, the reaction is carried out for a period of time comprised from 5-15 minutes, more preferably from 10-15 minutes.

The ion exchange step is carried out by reaction of the product obtained in step b) with a source of gold or silver in a suitable solvent, preferably water. In a preferred embodiment, the reaction is carried out under conditions that prevent the oxidation of silver or gold, such as e.g. light exposure. When the product of step b) is not isolated and the product is in solution, step c) may be carried out in the same solvent of step b).

The ion exchange step can be carried out in solution or by using ion exchange resins. In a preferred embodiment, the ion exchange is carried out in solution. Any gold or silver salt that is soluble in the solvent in which the ion exchange is carried out can be used as a source of gold or silver. In a particular embodiment, the silver salt is silver nitrate and the gold salt is gold chloride. In a preferred embodiment, the amount of source of gold or silver for carrying out the ion exchange is calculated by considering the content of metal ion (e.g., sodium, potassium, calcium, magnesium or zinc) in the starting high molecular weight glycosaminoglycan salt, and the content of gold or silver in the gold or silver salt used for step c). In a preferred embodiment, the amount of source of gold or silver of step c) is stoichiometric with respect to the high molecular weight glycosaminoglycan metal salt. Therefore, in this case, the metal content (%) in the starting high molecular weight glycosaminoglycan metal salt should be known.

For example, in the case of preparing silver chondroitin sulfate (Ag CS) from 2.5 g of sodium chondroitin sulfate (Na CS) containing 6% of sodium, and silver nitrate, the calculation would be made in the following manner:

% Na in Na CS used: 6%
% Ag necessary in exchange: 6%
% Ag in AgNO3: 63%

$$\frac{2.5\ g\ (Na\ CS) * 0.06\ g\ (of\ Na)}{0.63\ g} = 0.24\ g\ AgNO3$$

And in the case of preparing gold chondroitin sulfate (Au CS) from 2.5 g of sodium chondroitin sulfate (Na CS) containing 6% of sodium, and gold chloride, the calculation would be made in the following manner:

   % Au necessary in exchange: 6%
   % Au in gold chloride: 49%

$$\frac{2.5\ g\ (Na\ CS) * 0.06\ g\ (of\ Na)}{0.49\ g} = 0.31\ g\ gold\ chloride$$

In the case of using other gold or silver salts or other glycosaminoglycans the calculation would be made analogously.

The product of step c) can be optionally purified. Said purification may comprise the filtration of the solution obtained in step c) to remove solid impurities. Additionally or alternatively, the purification may include the precipitation of the product using organic solvents and isolation of the same, as previously described.

The optionally purified product of step c) can be dried and packaged in conditions that prevent the oxidation of silver or gold, e.g. avoiding exposure to light.

With respect to the specific conditions to carry out the process for the preparation of the partially depolymerized glycosaminoglycan gold or silver salts, the expert will know how to adjust the parameters of each of the steps of the process indicated previously in the light of the description and the examples of the present invention.

Moreover, the invention is also related to the products obtainable by the process described above. Thus, another aspect of the invention relates to a partially depolymerized glycosaminoglycan metal salt, wherein the metal is gold or silver, obtainable by a preparation process which comprises the steps a), a'), a"), b), and c) as previously defined.

The expression partially depolymerized glycosaminoglycan metal salt "obtainable by the process" of the invention is used herein for defining the partially depolymerized glycosaminoglycan metal salt by its preparation process and refers to the product that can be obtained through the preparation process which comprises the steps a), a'), a"), b), and c) as previously defined. For the purposes of the invention, the expressions "obtainable", "obtained" and similar equivalent expressions are used interchangeably and, in any case, the expression "obtainable" encompasses the expression "obtained".

Glycosaminoglycans (GAGs) are polysaccharides without ramifications that contain repetitions of one unit of disaccharides which comprises an acid sugar and an amino sugar. The amino sugar may be D-glucosamine (Glc) or D-galactosamine (Gal), wherein the amino group may be acetylated (Nac), and can also carry a sulfate group at the 4 or 6 carbon or at a non-acetylated nitrogen. The acid sugar may be D-glucuronic (GlcUA), L-iduronic (IDUA) acid or galactose (Gal).

Examples of glycosaminoglycans include chondroitin sulfate, hyaluronic acid, heparin, keratan sulfate, dermatan sulfate, and heparan sulfate.

Chondroitin sulfate (CS) is a glycosaminoglycan which comprises N-acetyl galactosamine (GalNac) and glucuronic acid (GlcUA) disaccharides. The hydroxyl groups at the positions 4 and 6 of the N-acetyl galactosamine may be sulfated, giving rise to two isomers: chondroitin 4-sulfate and chondroitin 6-sulfate.

Hyaluronic acid (HA) is a glycosaminoglycan which comprises N-acetyl glucosamine (GlcNac) and glucuronic acid (GlcUA) disaccharides.

Heparin is a glycosaminoglycan which comprises glucuronic acid (GlcUA) or L-iduronic acid (IdUA) and N-acetyl glucosamine (GlcNac) disaccharides. Heparan sulfate contains one disaccharide repetitive unit similar to the one of heparin but contains more N-acetyl groups, less N-sulfate groups and a lower degree of O-sulfate groups.

Keratan sulfate (KS) is a glycosaminoglycan which comprises galactose (Gal) and N-acetyl glucosamine (GlcNac) disaccharides. The content of sulfate is variable, an ester sulfate can be present at the 6 carbon both of the galactose and of the hexosamine. Two different types of keratan are known, keratan sulfate I, isolated from cornea, and keratan sulfate II, isolated from cartilage.

Dermatan sulfate (DS) is a glycosaminoglycan which comprises L-iduronic acid (IdUA) and N-acetyl galactosamine (GalNac) disaccharides.

The expression "partially depolymerized glycosaminoglycan" as used in this invention refers to the glycosaminoglycan that has been subjected to a depolymerization process such that the molecular weight of the product resulting from the depolymerization is lower than the molecular weight of the starting product. Preferably, the expression "partially depolymerized glycosaminoglycan" refers to the glycosaminoglycan that has been subjected to a depolymerization process which comprises the steps a), a'), and a") as described above. For example, in the case of chondroitin sulfate, the molecular weight of the high molecular weight chondroitin sulfate salt is comprised from 10.000-50.000 Daltons, while the partially depolymerized gold or silver chondroitin sulfate salt of the invention has a molecular weight comprised from 5.000-25.000 Daltons depending on the molecular weight of the starting compound and taking into account that the molecular weight of the partially depolymerized product will always be lower than the one of the non-depolymerized product. In the case of hyaluronic acid, the molecular weight of the high molecular weight hyaluronic acid salt is comprised from 50.000-2.500.000 Daltons, while the partially depolymerized gold or silver hyaluronate of the invention has a molecular weight comprised from 5.000-50.000 Daltons, depending on the molecular weight of the starting compound and taking into account that the molecular weight of the partially depolymerized product will always be lower than the one of the non-depolymerized product.

In a preferred embodiment, the glycosaminoglycan of the partially depolymerized glycosaminoglycan metal salt is selected from the group consisting of chondroitin sulfate, hyaluronic acid, heparin, heparan sulfate, keratan sulfate, and dermatan sulfate. In a more preferred embodiment, the glycosaminoglycan is chondroitin sulfate or hyaluronic acid.

In a preferred embodiment, the invention relates to a partially depolymerized glycosaminoglycan silver salt. In a more preferred embodiment, the glycosaminoglycan is selected from the group consisting of chondroitin sulfate, hyaluronic acid, heparin, heparan sulfate, keratan sulfate, and dermatan sulfate. In a still more preferred embodiment, the glycosaminoglycan is selected from the group consisting of chondroitin sulfate and hyaluronic acid.

In another preferred embodiment, the invention relates to a partially depolymerized glycosaminoglycan gold salt. In a more preferred embodiment, the glycosaminoglycan is selected from the group consisting of chondroitin sulfate, hyaluronic acid, heparin, heparan sulfate, keratan sulfate, and dermatan sulfate. In a still more preferred embodiment, the glycosaminoglycan is selected from the group consisting of chondroitin sulfate and hyaluronic acid.

In another preferred embodiment, the silver content by weight in the partially depolymerized glycosaminoglycan silver salt is comprised from 2-10%.

In another preferred embodiment, the content of gold by weight in the partially depolymerized glycosaminoglycan gold salt is comprised from 2-8%.

In another preferred embodiment, the invention relates to silver chondroitin sulfate, characterized by a specific rotation comprised from -12.0° to -30.0°; more preferably from -12.0° to -20.0°, from -20.0° to -30.0°, or from -15.0° to -25.0°, a silver content by weight comprised from 6-10%, a molecular weight comprised from 5.000-25.000 Daltons and a pH comprised from 5.5-7.5 when dissolved in water at 1 % by weight.

In another preferred embodiment, the invention relates to gold chondroitin sulfate, characterized by a specific rotation comprised from -12.0° to -30.0°; more preferably from -12.0° to -20.0°, from -20.0° to -30.0°, or from -15.0° to -25.0°, a content of gold by weight comprised from 6-10%, a molecular weight comprised from 5.000-25.000 Daltons and a pH comprised from 5.5-7.5 when dissolved in water at 1 % by weight.

In another preferred embodiment, the invention relates to silver hyaluronate, characterized by a silver content by weight comprised from 2-8%, a molecular weight comprised from 5.000-50.000 Daltons and a pH comprised from 5.0-8.0 when dissolved in water at 1 % by weight.

In another preferred embodiment, the invention relates to gold hyaluronate, characterized by a content of gold by weight comprised from 2-8%, a molecular weight comprised from 5.000-50.000 Daltons and a pH comprised from 5.0-8.0 when dissolved in water at 1 % by weight.

The partially depolymerized glycosaminoglycan metal salts of the present invention may form part of a pharmaceutical or cosmetic composition. Thus, another aspect of the invention relates to a pharmaceutical or cosmetic composition comprising an effective amount of the partially depolymerized glycosaminoglycan metal salt as defined above, together with one or more pharmaceutically or cosmetically acceptable excipients or carriers.

In a particular embodiment, the pharmaceutical or cosmetic composition of the invention is an oral composition.

In a preferred embodiment, the pharmaceutical or cosmetic composition of the invention is a topical composition.

In a particular embodiment, the composition further comprises arginine. In another particular embodiment, the composition consists of an effective amount of the partially depolymerized glycosaminoglycan metal salt as defined above, together with one or more excipients or carriers. In another particular embodiment, the composition consists of an effective amount of the partially depolymerized glycosaminoglycan metal salt as defined above and arginine together with one or more excipients or carriers.

The expression "effective amount" as used herein, relates to the amount of product that provides a cosmetic or therapeutic effect after its application. The effective amount that provides a therapeutic effect (also cited here as therapeutically effective amount) is the amount of a compound that, when administered, is sufficient to prevent the development of, or to relieve to some degree one or more of the symptoms of the disease to which it is directed. The particular dose of compound administered according to this invention may vary according to the particular conditions surrounding the case, including the administered compound, the route of administration, the particular condition being treated and similar considerations.

The expression "pharmaceutically acceptable excipients or carriers" means that the excipients or carriers are suitable for the preparation of compositions for pharmaceutical or medical uses. Each component must be pharmaceutically acceptable in the sense of being compatible with the other ingredients of the pharmaceutical composition. It must also be suitable for use in contact with tissues or organs of humans and animals without excessive toxicity, irritation, allergic response, immunogenicity or other problems or complications consistent with a reasonable risk/benefit relationship.

The expression "cosmetically acceptable excipients or carriers" means that the excipients or carriers are suitable for the preparation of compositions for cosmetic use. Each component must be cosmetically acceptable in the sense of being compatible with the other ingredients of the cosmetic composition. It must also be suitable for use in contact with tissues or organs of humans and animals without excessive toxicity, irritation, allergic response, immunogenicity or other problems or complications consistent with a reasonable risk/benefit relationship.

The topical formulations of the invention can be directly applied on the skin or by other methods, e.g. encapsulated in a matrix sensitive to temperature and/or pressure, in film or soluble solid vehicle in the body fluids, and the like.

Examples of topical compositions include creams, gels, hydrogels, dressings, shampoos, dyes, pastes, ointments, pomades, powders, liquid or semi-liquid formulations and similar compositions. The application of said compositions may be done by means of aerosol e.g. with a propellant, or without propellant such as in the case of a pump sprayer, or in the form of drops, lotions or as a semi-solid. Moreover, plasters, bandages, gauze pads and similar coverings, containing a suitable amount of an active ingredient, may also be used. In a particular embodiment, the topical composition is in powder or hydrogel form.

In a particular embodiment, the topical composition is a pharmaceutical composition which comprises a therapeutically effective amount of the partially depolymerized glycosaminoglycan metal salt as previously defined, together with one or more pharmaceutically acceptable excipients or carriers.

In another particular embodiment, the topical composition is a cosmetic composition which comprises an effective amount of the partially depolymerized glycosaminoglycan metal salt as previously defined, together with one or more cosmetically acceptable excipients or carriers.

The topical compositions defined above comprise pharmaceutical or cosmetic excipients or carriers appropriate for topical administration, including, moisturizing, emollient, emulsifying, thickening, humectant, pH regulators, antioxidant, preservative agents, or mixtures thereof. The excipients or carriers used have affinity for the skin, are well tolerated, are stable, and are used in an amount suitable to provide the desired consistency and ease of application.

Although the invention is especially related to topical and oral formulations also form part of the present invention other applications of the salts as described above such as buccal, otic, ocular, ophthalmic and injectable administration. Moreover, this invention is also related to compositions of the salts as described above for application in medical devices or for incorporation into a coating composition.

The salts of the present invention are useful in the treatment of wounds, scars and burns. They are also useful in the treatment and/or prevention of microbial infections and inflammatory processes of the skin, such as e.g. acne and psoriasis.

Therefore, another aspect of the present invention relates to the use of the partially depolymerized glycosaminoglycan metal salts as described above for the preparation of a medicament for the treatment of wounds, scars and burns. Said aspect may also be formulated as the partially depolymerized glycosaminoglycan metal salts as described above for use in the treatment of wounds, scars and burns. It is also part of the disclosure a method for the treatment of wounds, scars and burns, which comprises administering a therapeutically effective amount of a partially depolymerized glycosaminoglycan metal salt as described above, together with pharmaceutically acceptable excipients or carriers, in a patient, including a human.

Another aspect of the present invention relates to the use of the partially depolymerized glycosaminoglycan metal salts as described above for the preparation of a medicament for the treatment and/or prevention of microbial infections and inflammatory processes of the skin, including acne and psoriasis. Said aspect may also be formulated as the partially depolymerized glycosaminoglycan metal salts as described above for use in the treatment and/or prevention of microbial infections and inflammatory processes of the skin, including acne and psoriasis. It is also part of the invention a method for the treatment and/or prevention of microbial infections and inflammatory processes of the skin, including acne and psoriasis, which comprises administering a therapeutically effective amount of a partially depolymerized glycosaminoglycan metal salt as described above, together with pharmaceutically acceptable excipients or carriers, in a patient, including a human.

The salts of the present invention are also useful in the cosmetic field for skin care, in particular as moisturizing agents and as skin regenerating agents. In a particular embodiment, the skin care comprises the improvement of at least one of the following symptoms: roughness, flakiness, tightness, dryness of the skin, chapping, lack of elasticity and aging.

In a particular embodiment, the salts of the invention are used together with arginine in the pharmaceutical and cosmetic uses indicated above.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

EXAMPLES

Example 1: Partially depolymerized silver chondroitin sulfate

Depolymerization with $KMnO_4$

Sodium chondroitin sulfate having a molecular weight comprised from 25.000 and 40.000 Daltons (2.5 g) was dissolved in water (25 mL) under stirring. The total volume was measured and the pH was adjusted to 8.5 with NaOH 30% (weight/volume). Once the pH was adjusted, it was heated to a temperature of 85 °C. $KMnO_4$ was added slowly under stirring (20% by weight with respect to the amount of starting glycosaminoglycan). It was maintained under stirring at this temperature for 1 hour. The solution was filtered through a filter of 0.8 microns.

Depolymerization with NaOH

The pH of the solution obtained in the previous step was adjusted at 11 with NaOH 30% (weight/volume). The volume of the solution obtained was measured and 1.5% of NaCl (on the total volume) was added under stirring until dissolution. The temperature was adjusted at 45 °C. It was maintained under stirring at this temperature for 12 hours.

Depolymerization with $H_2O_2$

The solution obtained in the previous step was allowed to cool down until reaching a temperature of 25 °C and the total volume was measured. It was checked that the pH was between 10-11. 1.5% (weight/volume) of $H_2O_2$, on the total volume, was added slowly under stirring. It was maintained under stirring at this temperature for 1 hour. The solution was filtered through a filter of 0.8 microns. The pH was adjusted to 6 with acetic acid (filtered liquid).

Precipitation with solvents

The total volume of the filtered liquid obtained in the previous step was measured. 3 volumes of ethanol (on the volume previously measured) were added slowly under stirring. It was stirred for 15 minutes. Stirring was stopped and allowed to rest 3 h (up to complete precipitation).

Bleaching

The precipitate obtained was separated by decantation ensuring the maximum removal of the residual solvent. Once the precipitate was separated, it was dissolved with the same amount of water as in the initial step of dissolution. It was stirred until complete dissolution. Once dissolved, $H_2O_2$ 30% (weight/volume) was added slowly under stirring. The amount added was 50% by weight of the amount by weight of glycosaminoglycan initially used (1.25 g $H_2O_2$). It was stirred at room temperature for 10 minutes.

Ion exchange with silver salts

0.24 g of silver nitrate was added slowly under stirring. It was stirred 5 minutes (until complete dissolution and homogenization).

Precipitation with solvents

The total volume of the solution obtained in the previous step was measured. 3 volumes of ethanol (on the volume previously measured) were added slowly under stirring. It was allowed to stir for 15 minutes. Stirring was stopped and it was allowed to rest 3 h.

Dehydration and drying

The precipitate was separated by decantation ensuring the maximum possible removal of the residual solvent. Once the precipitate was separated, ethanol was added over the precipitate. The amount to be added was: amount of glycosaminoglycan initially used x 6 = amount of solvent to be added. It was stirred vigorously until complete homogenization. It was stirred for 20 minutes. Stirring was stopped and it was allowed to rest 2 h. The precipitate was separated by decanting and subsequently filtered ensurng the maximum removal of the residual solvent. Once the precipitate was separated, ethanol was added over the precipitate. The amount added was: amount of glycosaminoglycan initially used x 6 = amount of solvent added. It was stirred vigorously until complete homogenization. It was stirred for 20 minutes. Stirring was stopped and it was allowed to rest a minimum of 2 h. The precipitate was separated by filtration. The maximum removal of the residual solvent was verified. Once the precipitate was separated, it was dried in an oven at a temperature of 75 °C until the moisture of the product was lower than 10 % and it was packaged in double bag.

Example 2: Partially depolymerized silver hyaluronate

The desired product was obtained analogously to the process described in example 1, starting from a sodium hyaluronate solution having a molecular weight higher than 1.000.000 Daltons (2 g) in water (145 mL). The amount added in the ion exchange step was 0.13 g of silver nitrate.

Example 3: Partially depolymerized gold chondroitin sulfate

The desired product was obtained analogously to the process described in example 1, starting from a solution of 1 g of sodium chondroitin sulfate having a molecular weight comprised from 25.000 and 40.000 Da in water (10 mL) and replacing silver nitrate by gold chloride (0.12 g).

Example 4: Partially depolymerized gold hyaluronate

The desired product was obtained analogously to the process described in example 1, starting from a sodium hyaluronate solution having a molecular weight higher than 1.000.000 Daltons (2 g) in water (145 mL) and replacing silver nitrate by gold chloride (0.16 g).
Comparative example 5: Partially depolymerized silver chondroitin sulfate

(bleaching agent: sodium metabisulfite)

The desired product was obtained analogously to the process described in example 1, starting from a solution of 1 g of sodium chondroitin sulfate having a molecular weight comprised from 25.000 and 40.000 Da in water (10 mL) and replacing the hydrogen peroxide of the bleaching step by sodium metabisulfite (0.5 g).

Comparative example 6: Partially depolymerized silver chondroitin sulfate (bleaching agent: sodium thiosulfate)

The desired product was obtained analogously to the process described in example 1, starting from a solution of 1 g of sodium chondroitin sulfate having a molecular weight comprised from 25.000 and 40.000 Da in water (10 mL) and replacing the hydrogen peroxide of the bleaching step by sodium thiosulfate (0.1 g).

Comparative example 7: Partially depolymerized silver chondroitin sulfate (without bleaching step)

The desired product was obtained analogously to the process described in example 1, but carrying out the depolymerization step with $H_2O_2$ at pH 7 and without carrying out the bleaching step.

Comparative example 8: High molecular weight silver chondroitin sulfate

Sodium chondroitin sulfate having a molecular weight between 25.000 and 40.000 Da (1 g) was dissolved in water (10 mL) and under stirring. The total volume was measured and the pH was adjusted to 6 with NaOH 30% (weight/volume). 0.1 g of silver nitrate was added slowly under stirring. It was stirred 5 minutes (until complete dissolution and homogenization). The total volume was measured. 2 volumes of ethanol (on the volume previously measured) were added slowly under stirring. It was allowed to stir for 10 minutes. Stirring was stopped and it was allowed to rest 3 h.

Comparative example 9: High molecular weight silver hyaluronate

Sodium hyaluronate having a molecular weight higher than 1.000.000 Daltons (2 g) was dissolved in water (145 mL) and under stirring. The total volume was measured and the pH was adjusted to 6 with NaOH 30% (weight/volume). 0.1 g of silver nitrate was added slowly under stirring. It was stirred for 5 minutes (until complete dissolution and homogenization). The total volume was measured. 2 volumes of ethanol (on the volume previously measured) were added slowly under stirring. It was allowed to stir for 10 minutes. Stirring was stopped and it was allowed to rest 3 h.

Stability test

The precipitated products obtained in the example 1 and the comparative examples 5-8, were protected in the dark (inside of a closet) for 1 hour. After this time, a visual inspection of the products was carried out and the products were protected in the dark again. After 12 hours, another visual inspection of the products was carried out. The products were then exposed to ambient light. After 5 hours, a visual inspection of the products was carried out.

The results showed that for the product of example 1, which included the depolymerization step and the bleaching step with hydrogen peroxide, the color was white both after 1 hour in the dark and after 12 hours in the dark, and it remained white after 5 hours of exposure to ambient light. In the case of the product of the comparative example 5, which included the depolymerization step and the bleaching step with sodium metabisulfite, the color was reddish after 1 hour in the dark, reddish to black after 12 hours in the dark, and black after 5 hours of exposure to ambient light. In the case of the product of the comparative example 6, which included the depolymerization step and the bleaching step with sodium thiosulfite, the color was black both after 1 hour in the dark and after 12 hours in the dark, and it remained black after 5 hours of exposure to ambient light. In the case of the product of the comparative example 7, which included the depolymerization step but not the bleaching step, the color was white after 1 hour in the dark and after 12 hours in the dark, but it was dark red after 5 hours of exposure to ambient light. Finally, for the product of the comparative example 8, which did not include the depolymerization step nor the bleaching step, the color was white after 1 hour in the dark and after 12 hours in the dark, but it was dark red after 5 hours of exposure to ambient light.

The results obtained indicate that the product of the invention (example 1) has an important improvement in stability since it stays stable for 5 h when exposed to ambient light, in particular, in comparison with the product of the state of the art described in CN 1687141 (comparative example 8). On the other hand, it is observed that when any of the conditions of the process for the preparation of the partially depolymerized salts (such as e.g. when changing the bleaching agent or when the bleaching step is not performed) is varied, the resulting product is not stable to light in a period of 5 h.

Test of antimicrobial activity

The objective of the test was to verify the antimicrobial activity with the microorganisms *E. Coli* and *S. Aureus.* For this purpose, solutions in water of the following products were separately prepared:

- Commercially available sodium chondroitin sulfate of marine origin having a molecular weight between 25.000 and 40.000 Daltons. Product described in European Pharmacopeia (01/2009:2064) (comparative example 10);
- High molecular weight silver chondroitin sulfate (comparative example 8);
- Partially depolymerized silver chondroitin sulfate (example 1);
- High molecular weight silver hyaluronate (comparative example 9);
- Partially depolymerized silver hyaluronate (example 2);
- $AgNO_3$ (comparative example 11);
- Partially depolymerized gold chondroitin sulfate (example 3);
- Partially depolymerized gold hyaluronate (example 4); and
- Mixture of 1 g of commercially available sodium chondroitin sulfate of marine origin having a molecular weight between 25.000 and 40.000 Daltons and 0.1 g of $AgNO_3$ (comparative example 12).

The products derived from chondroitin sulfate were dissolved at 10% (weight/volume). The products derived from hyaluronic acid were dissolved at 1.4% (weight/volume). For the rest of products water was added until complete dissolution.

Each of the products was applied, as well as a control (water), to a plate with *S. Aureus* and *E. Coli.* The products were incubated under standard conditions for *S. Aureus* and *E. Coli.* The results were expressed in areolar or halo diameter and were the following: for the comparative example 10 it was 0 in *E. Coli* and 0 in *S. Aureus;* for the comparative example 8 it was 7 in *E. Coli* and 8 in *S. Aureus;* for the example 1 it was 7 in *E. Coli* and 6 in *S. Aureus;* for the comparative example 9 it was 8 in *E. Coli* and 8 in *S. Aureus;* for the example 2 it was 7 in *E. Coli* and 6 in *S.*

*Aureus;* for the comparative example 11 it was 8 in *E. Coli* and 6 in *S. Aureus;* for the example 3 it was 9 in *E. Coli* and 8 in *S. Aureus;* for the control it was 0 in *E. Coli* and 0 in *S. Aureus;* for the example 4 it was 4 in *E. Coli* and 6 in *S. Aureus;* and for the comparative example 12 it was 7 in *E. Coli* and 5 in *S. Aureus.*

The results obtained confirmed that the products of the invention with gold (examples 3 and 4) and silver (examples 1 and 2) have an antimicrobial activity similar to silver nitrate.

Preliminary test of toxicology and effectiveness in burns

The aim of this test was to assess the toxicity (topical) and effectiveness in burns. The side effects that the different products could cause including irritation, inflammation and/or darkening were assessed by visual inspection and photographs. The products assessed were the following:

- Commercially available sodium chondroitin sulfate of marine origin having a molecular weight between 25.000 and 40.000 Daltons. Product described in European Pharmacopeia (01/2009:2064) (comparative example 10);
- Partially depolymerized silver chondroitin sulfate (example 1);
- Partially depolymerized silver chondroitin sulfate dissolved/mixed in a moisturizing cream (example 1c);
- Partially depolymerized silver hyaluronate (example 2);
- AgNO$_3$ (comparative example 11); and
- Partially depolymerized gold chondroitin sulfate (example 3).

The products derived from chondroitin sulfate were dissolved in water at 10% (weight/volume). The products derived from hyaluronic acid were dissolved at 1.4% (weight/volume). For the silver nitrate water was added until complete dissolution. The product of example 1c was mixed with a moisturizing cream.

All the products were applied in the "upper area of the arm" (upper biceps) and in the abdomen (area of the ribs). The products of examples 1, 2 and 3 were also applied in the "lower area of the arm". The visual inspections were carried out with the following frequency after the application: 10 minutes, 30 minutes, 60 minutes, 8 h, 3 days, 4 days, 7 days and 9 days.

The treated areas were exposed to different types of light (solar, incandescent bulb, fluorescent) for 10 minutes. Darkening was not observed in any case. The abdomen was protected from the light after 10 minutes. The "upper area of the arm" was left under light exposure up to 8 hours. The "lower area of the arm" had continuous exposure to ambient light.

According to the results obtained, in the application of the product of the comparative example 10, a caramelized appearance was observed both in the "upper area of the arm" and in the abdomen at 10 minutes and at 30 minutes. In the controls carried out at 60 minutes, 8 hours, 24 hours, 48 hours, 3 days, 4 days, 7 days and 9 days, no relevant results were observed in any of the parts ("upper area of the arm"/abdomen).

The application of the product of examples 1, 1c, 2 and 3 showed no relevant behavior in any of the areas where the product was applied in any of the inspections carried out at 10 minutes, 30 minutes, 60 minutes, 8 hours, 24 hours, 48 hours, 3 days, 4 days, 7 days and 9 days.

In the application of the product of the comparative example 11 irritation was observed both in the "upper area of the arm" and in the abdomen at 10 minutes and at 30 minutes. After 60 minutes, there was irritation, itching and burning in the parts where the product was applied. After 8 hours, there was irritation, itching, burning and a halo or aureole. After the third day, an increase of the diameter of the aureole of the burn was observed. At this moment, the product of example 1 was applied to the burn of the abdomen. 2 daily applications of the product of example 1 were done to the burn during the next 3 days. After the fourth day an increase of the diameter of the aureole of the burn was observed in the "upper area of the arm" and a nearly full disappearance of the burn treated with example 1 in the abdomen. After the seventh day the diameter of the aureole of the burn increased in the "upper area of the arm". At this moment, the product of example 2 was applied to the burn of the "upper area of the arm". 3 daily applications of the product of example 2 were done to the burn. After the ninth day, the burn treated with the product of example 2 of the "upper area of the arm" had almost completely disappeared.

In agreement with the results obtained, and unlike silver nitrate, the products of examples 1, 1c, 2 and 3 of the invention did not have problems of darkening, irritation, itching or inflammation. Moreover, the tests of efficacy carried out with the products of examples 1 and 2 of the invention confirmed that they produce an improvement in the treatment of skin surfaces with burns or wounds.

Acute oral toxicology test

An acute oral toxicology test of partially depolymerized silver chondroitin sulfate (example 1) was carried out to assess the oral toxicity and identify the corresponding classification (GHS, Globally Harmonised Classification System for Chemical Substances and Mixtures). The study was conducted according to the OECD Guideline No. 420 (fixed dose). The results interpreted according to this guideline indicated that the product partially depolymerized silver chondroitin sulfate had a median lethal oral dose (LD50)> 2000/kg and can be included in the GHS classification (Globally Harmonised Classification System for Chemical Substances and Mixtures) 5, i.e. in the group having less toxicity.

Absorption test

A comparative test of the absorption of the depolymerized compounds of the invention and the non-depolymerized compounds was carried out. To do this, partially depolymerized silver chondroitin sulfate (example 1) and high molecular weight silver chondroitin sulfate (comparative example 8) were dissolved separately with water at the same concentration (10% by weight/volume). Once the products were completely dissolved it was noted that the viscosity of the product of example 1 was lower than that of the product of the comparative example 8. 10 mL of each product were applied on cellulose paper. It was noted that the product of example 1 was absorbed more rapidly than the product of the comparative example 8. Taking into account that, for the purposes of absorption, the behavior on cellulose paper "may be extrapolated" to organic tissues (skin), with this result, it is concluded that the depolymerized product of the invention has a better absorption than the non-depolymerized product.

Viscosity Test

The objective of the test was to verify that the viscosity of the partially depolymerized products obtained is lower than the viscosity of the starting products (non-depolymerized). The products assessed were the following:

- High molecular weight silver chondroitin sulfate (comparative example 8);
- Partially depolymerized silver chondroitin sulfate (example 1);
- High molecular weight silver hyaluronate (comparative example 9) and
- Partially depolymerized silver hyaluronate (example 2).

The following parameters were assessed: dissolution time, appearance (visual method) and displacement time.
0.01 g of each product was dissolved separately in 0.5 mL of distilled water in test tubes of 11.5 cm of length and 1 cm of diameter. Once the products were completely dissolved, the position of the tubes was inverted to check the displacement time.
Dissolution control: The partially depolymerized products were dissolved in less than 10 seconds, while the non-depolymerized products took more than 3 minutes to dissolve. Therefore, it is concluded that the dissolution of the partially depolymerized products is faster than that of the non-depolymerized products, which gives the partially depolymerized products the advantage of being more easily applicable and manipulated.
Appearance control: when inverting the position of the tubes (180° rotation), it was visually appreciated that the viscosity of the solutions of the partially depolymerized products was clearly lower than that of the solutions of the non-depolymerized products.
Displacement control: when inverting the position of the tubes (180° rotation), the displacement time was measured with the following results: the displacement time of the comparative example 8 was 30 seconds; the displacement time of example 1 was 1 second; the displacement time of the comparative example 9 was 5 minutes; and finally, the displacement time of example 2 was 1 second.
It is concluded in a qualitative manner that the molecular weights of the partially depolymerized products are substantially lower than the non-depolymerized products, considering the relationship between viscosity and molecular weight. One of the methods used for the determination having a molecular weight of glycosaminoglycans is by means of the verification of the intrinsic viscosity and determination of the molecular weight by means of the Mark-Houwink constant/ratio.

CITED REFERENCES

- US 2010317617
- CN 1687141
- EP 1878754
- WO 8705517

Dissolution control: The partially depolymerized products were dissolved in less than 10 seconds, while the non-depolymerized products took more than 3 minutes to dissolve. Therefore, it is concluded that the dissolution of the partially depolymerized products is faster than that of the non-depolymerized products, which gives the partially depolymerized products the advantage of being more easily applicable and manipulated.

Appearance control: when inverting the position of the tubes (180° rotation), it was visually appreciated that the viscosity of the solutions of the partially depolymerized products was clearly lower than that of the solutions of the non-depolymerized products.

Displacement control: when inverting the position of the tubes (180° rotation), the displacement time was measured with the following results: the displacement time of the comparative example 8 was 30 seconds; the displacement time of example 1 was 1 second; the displacement time of the comparative example 9 was 5 minutes; and finally, the displacement time of example 2 was 1 second.

It is concluded in a qualitative manner that the molecular weights of the partially depolymerized products are substantially lower than the non-depolymerized products, considering the relationship between viscosity and molecular weight. One of the methods used for the determination having a molecular weight of glycosaminoglycans is by means of the verification of the intrinsic viscosity and determination of the molecular weight by means of the Mark-Houwink constant/ratio.

CITED REFERENCES

- US 2010317617
- CN 1687141
- EP 1878754
- WO 8705517

**Claims**

1. Process for the preparation of a partially depolymerized glycosaminoglycan metal salt, wherein the metal is gold or silver, which comprises the following steps:

   a) depolymerization of a high molecular weight glycosaminoglycan metal salt; wherein the metal is different from gold or silver, with a depolymerizing agent selected from the group consisting of potassium permanganate, sodium hydroxide, and hydrogen peroxide;
   a') depolymerization of the product obtained in step a) with a depolymerizing agent selected from the group consisting of potassium permanganate, sodium hydroxide, and hydrogen peroxide;
   a") depolymerization of the product obtained in step a') with a depolymerizing agent selected from the group consisting of potassium permanganate, sodium hydroxide, and hydrogen peroxide;
   wherein the depolymerizing agent of step a) is potassium permanganate, the depolymerizing agent of step a') is sodium hydroxide, and the depolymerizing agent of step a") is hydrogen peroxide;
   b) bleaching of the product obtained in the previous step with hydrogen peroxide; and
   c) metal ion exchange of the product obtained in the previous step for gold or silver by using a source of gold or silver.

2. Process according to claim 1, wherein the amount of potassium permanganate is comprised from 5-30% by weight of the amount by weight of high molecular weight glycosaminoglycan metal salt.

3. Process according to any of the claims 1-2, wherein the depolymerization with sodium hydroxide is carried out in the presence of sodium chloride in an amount comprised from 10-20% by weight of the amount by weight of high molecular weight glycosaminoglycan metal salt.

4. Process according to any of the claims 1-3, wherein in the depolymerization step with hydrogen peroxide the amount of hydrogen peroxide is comprised from 5-25% by weight of the amount by weight of high molecular weight glycosaminoglycan metal salt.

5. Partially depolymerized glycosaminoglycan metal salt, wherein the metal is gold or silver, obtainable by the preparation process according to any of the claims 1-4.

6. Glycosaminoglycan metal salt according to claim 5, wherein the glycosaminoglycan is selected from the group consisting of chondroitin sulfate, hyaluronic acid, heparin, heparan sulfate, keratan sulfate and dermatan sulfate.

7. Pharmaceutical or cosmetic composition comprising an effective amount of the partially depolymerized glycosaminoglycan metal salt defined in any of the claims 5-6, together with one or more pharmaceutically or cosmetically acceptable excipients or carriers.

8. Pharmaceutical or cosmetic composition according to claim 7, which is an oral composition or a topical composition.

9. Pharmaceutical or cosmetic composition, which is a topical composition according to claim 8, in powder or hydrogel form.

10. Pharmaceutical or cosmetic composition according to any of the claims 7-9, further comprising arginine.

11. Partially depolymerized glycosaminoglycan metal salts as defined in any of the claims 5-6, for use in the treatment of wounds, scars and burns.

12. Partially depolymerized glycosaminoglycan metal salts as defined in any of the claims 5-6, for use in the treatment and/or prevention of microbial infections and inflammatory processes of the skin.

13. Partially depolymerized glycosaminoglycan metal salts for use according to claim 12, wherein the inflammatory process of the skin is acne or psoriasis.

14. Use of the topical cosmetic composition defined in any of the claims 9-10, for non-therapeutic skin care.

15. Use according to claim 14, wherein the skin care comprises the improvement of at least one of the following symptoms: roughness, flakiness, tightness, dryness, chapping, lack of elasticity and aging.


**Patentansprüche**

1. Verfahren zur Herstellung von einem teilweise depolymerisierten Metallsalz von Glycosaminoglycan, wobei das Metall Gold oder Silber ist, welches folgende Schritte umfasst:

   a) Depolymerisation eines Metallsalzes hohen Molekulargewichts von Glycosaminoglycan; wobei das Metall nicht Gold oder Silber ist, mit einem Depolymerisationsmittel ausgewählt aus der Gruppe bestehend aus Kaliumpermanganat, Natriumhydroxid, und Wasserstoffperoxid;
   a') Depolimerisation des im Schritt a) erhaltenen Produkts mit einem Depolymerisationsmittel ausgewählt aus der Gruppe bestehend aus Kaliumpermanganat, Natriumhydroxid, und Wasserstoffperoxid;
   a") Depolimerisation des im Schritt a') erhaltenen Produkts mit einem Depolymerisationsmittel ausgewählt aus der Gruppe bestehend aus Kaliumpermanganat, Natriumhydroxid, und Wasserstoffperoxid;
   wobei das Depolymerisationsmittel des Schritts a) Kaliumpermanganat ist, das Depolymerisationsmittel des Schritts a') Natriumhydroxid ist, und das Depolymerisationsmittel des Schritts a") Wasserstoffperoxid ist;
   b) Bleichen des im vorherigen Schritt erhaltenen Produkts mit Wasserstoffperoxid; und
   c) Metallionenaustausch des im vorherigen Schritt erhaltenen Produkts mit Gold oder Silber unter Verwendung von einer Gold- bzw. Silberquelle.

2. Verfahren nach Anspruch 1, wobei der Gehalt an Kaliumpermanganat von 5-30 Gew.-% bezogen auf den Gewichtsanteil des Metallsalzes hohen Molekulargewichts von Glycosaminoglycan reicht.

3. Verfahren nach einem der Ansprüche 1-2, wobei die Depolymerisation mit Natriumhydroxid in Gegenwart von Natriumchlorid in einer Menge von 10-20 Gew.-% bezogen auf den Gewichtsanteil des Metallsalzes hohen Molekulargewichts von Glycosaminoglycan durchgeführt wird.

4. Verfahren nach einem der Ansprüche 1-3, wobei der Gehalt an Wasserstoffperoxid im Depolymerisationsschritt mit Wasserstoffperoxid von 5-25 Gew.-% bezogen auf den Gewichtsanteil des Metallsalzes hohen Molekulargewichts von Glycosaminoglycan reicht.

**5.** Teilweise depolymerisiertes Metallsalz von Glycosaminoglycan, wobei das Metall Gold oder Silber ist, welches mittels des Herstellungsverfahrens nach einem der Ansprüche 1-4 erhalten werden kann.

**6.** Metallsalz von Glycosaminoglycan nach Anspruch 5, wobei das Glycosaminoglycan ausgewählt aus der Gruppe bestehend aus Chondroitinsulfat, Hyaluronsäure, Heparin, Heparansulfat, Keratansulfat und Dermatansulfat ist.

**7.** Pharmazeutische oder kosmetische Zusammensetzung umfassend eine wirksame Menge des in einem der Ansprüche 5-6 definierten teilweise depolymerisierten Metallsalzes von Glycosaminoglycan zusammen mit einem oder mehreren pharmazeutisch oder kosmetisch akzeptablen Hilfsstoffen oder Trägerstoffen.

**8.** Pharmazeutische oder kosmetische Zusammensetzung nach Anspruch 7, welche eine orale Zusammensetzung oder eine topische Zusammensetzung ist.

**9.** Pharmazeutische oder kosmetische Zusammensetzung, welche eine topische Zusammensetzung nach Anspruch 8 ist, in Form eines Pulvers oder eines Hydrogels.

**10.** Pharmazeutische oder kosmetische Zusammensetzung nach einem der Ansprüche 7-9, weiterhin umfassend Arginin.

**11.** Teilweise depolymerisierte Metallsalze von Glycosaminoglycan wie in einem der Ansprüche 5-6 definiert, zur Verwendung in der Behandlung von Wunden, Narben oder Brandwunden.

**12.** Teilweise depolymerisierte Metallsalze von Glycosaminoglycan wie in einem der Ansprüche 5-6 definiert, zur Verwendung in der Behandlung und/oder Prävention von mikrobiellen Infektionen und Entzündungsprozessen der Haut.

**13.** Teilweise depolymeriserte Metallsalze von Glycosaminoglycan zur Verwendung nach Anspruch 12, wobei das Entzündungsprozess der Haut Akne oder Psoriasis ist.

**14.** Verwendung der in einem der Ansprüche 9-10 definierten topischen kosmetischen Zusammensetzung zur nicht therapeutischen Hautpflege.

**15.** Verwendung nach Anspruch 14, wobei die Hautpflege die Besserung von mindestens einem der folgenden Symptomen umfasst: Rauheit, Schuppenbildung, Straffung, Trockenheit, Entstehung von Rissen, mangelnder Elastizität und Alterung.

**Revendications**

**1.** Procédé de préparation d'un sel de métal de gycosaminoglycane partiellement dépolymérisé, dans lequel le métal est l'or ou l'argent, qui comprend les étapes suivantes:

a) dépolymérisation d'un sel de métal de glycosaminoglycane de haute masse moléculaire; où le métal est différent de l'or ou de l'argent, avec un agent de dépolymérisation choisi dans le groupe constitué du permanganate de potassium, de l'hydroxyde de sodium et du peroxyde d'hydrogène;
a') dépolymérisation du produit obtenu dans l'étape a) avec un agent de dépolymérisation choisi dans le groupe constitué du permanganate de potassium, de l'hydroxyde de sodium, et du peroxyde d'hydrogène;
a") dépolymérisation du produit obtenu dans l'étape a') avec un agent de dépolymérisation choisi dans le groupe constitué du permanganate de potassium, de l'hydroxyde de sodium, et du peroxyde d'hydrogène;
dans lequel l'agent de dépolymérisation de l'étape a) est le permanganate de potassium, l'agent de dépolymérisation de l'étape a') est l'hydroxyde de sodium, et l'agent de dépolymérisation de l'étape a") est le péroxyde d'hydrogène;
b) blanchissement du produit obtenu dans l'étape précédente avec du péroxyde d'hydrogène; et
c) échange d'ions de métal du produit obtenu dans l'étape précédente avec de l'or ou de l'argent en utilisant une source d'or ou d'argent.

**2.** Procédé selon la revendication 1, dans lequel la quantité de permanganate de potassium est comprise de 5-30 % en poids de la quantité en poids de sel de métal de glycosaminoglycane de haute masse moléculaire.

**3.** Procédé de l'une quelconque des revendications 1-2, dans lequel la dépolymérisation avec de l'hydroxyde de sodium est effectuée en présence de chlorure de sodium dans une quantité allant de 10-20 % en poids de la quantité en poids de sel de métal de glycosaminoglycane de haute masse moléculaire.

**4.** Procédé selon l'une quelconque des revendications 1-3, dans lequel dans l'étape de dépolymérisation avec du péroxyde d'hydrogène la quantité de péroxyde d'hydrogène est comprise de 5-25% en poids de la quantité en poids de sel de métal de glycosaminoglycane de haute masse moléculaire.

**5.** Sel de métal de glycosaminoglycane partiellement dépolymérisé, dans lequel le métal est l'or ou l'argent, qui peut être obtenu moyennant le procédé de préparation selon l'une quelconque des revendications 1-4.

**6.** Sel de métal de glycosaminoglycane selon la revendication 5, dans lequel le glycosaminoglycane est choisi dans le groupe constitué du sulfate de chondroïtine, de l'acide hyaluronique, de l'héparine, du sulfate d'héparane, du sulfate de kératane et du sulfate de dermatane.

**7.** Composition pharmaceutique ou cosmétique comprenant une quantité efficace du sel de métal de glycosaminoglycane partiellement dépolymérisé défini dans l'une quelconque des revendications 5-6, avec un ou plusieurs excipients ou véhicules pharmaceutiquement ou cosmétiquement acceptables.

**8.** Composition pharmaceutique ou cosmétique selon la revendication 7, qui est une composition orale ou une composition topique.

**9.** Composition pharmaceutique ou cosmétique, qui est une composition topique selon la revendication 8, sous forme de poudre ou d'hydrogel.

**10.** Composition pharmaceutique ou cosmétique selon l'une quelconque des revendications 7-9, comprenant en outre de l'arginine.

**11.** Des sels de métal de glycosaminoglycane partiellement dépolymérisés tels que définis dans l'une quelconque des revendications 5-6, pour l'utilisation dans le traitement de blessures, de cicatrices et de brûlures.

**12.** Des sels de métal de glycosaminoglycane partiellement dépolymérisés tels que définis dans l'une quelconque des revendications 5-6, pour l'utilisation dans le traitement et/ou la prévention d'infections microbiennes et de processus inflammatoires de la peau.

**13.** Des sels de métal de glycosaminoglycane partiellement dépolymérisés pour l'utilisation selon la revendication 12, où le processus inflammatoire de la peau est l'acné ou le psoriasis.

**14.** Utilisation de la composition cosmétique topique définie dans l'une quelconque des revendications 9-10, pour le soin non thérapeutique de la peau.

**15.** Utilisation selon la revendication 14, où le soin de la peau comprend l'amélioration d'au moins un des symptômes suivants: la rugosité, les squames cutanées, le tiraillement, la sécheresse, les gerçures, la manque d'élasticité et le vieillissement.

EP 2 814 848 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010317617 A
- WO 8705517 A
- CN 1687141
- EP 1878754 A